(19) Europäisches Patentamt
European Patent Office
Office européen des brevets

(11) **EP 1 511 421 B1**

(12) **FASCICULE DE BREVET EUROPEEN**

(45) Date de publication et mention
de la délivrance du brevet:
**19.09.2012 Bulletin 2012/38**

(21) Numéro de dépôt: **03755198.3**

(22) Date de dépôt: **23.05.2003**

(51) Int Cl.:
*A61B 6/02* *(2006.01)*     *A61B 6/00* *(2006.01)*

(86) Numéro de dépôt international:
**PCT/FR2003/001576**

(87) Numéro de publication internationale:
**WO 2003/099124 (04.12.2003 Gazette 2003/49)**

(54) **DISPOSITIF DE STEREORADIOGRAPHIE ET PROCEDURE D UTILISATION**

STEREORADIOGRAPHIE-VORRICHTUNG UND ANWENDUNGSVERFAHREN DAFÜR

STEREORADIOGRAPHY DEVICE AND METHOD FOR THE USE THEREOF

(84) Etats contractants désignés:
**AT BE BG CH CY CZ DE DK EE ES FI FR GB GR HU IE IT LI LU MC NL PT RO SE SI SK TR**

(30) Priorité: **23.05.2002 US 382372 P**

(43) Date de publication de la demande:
**09.03.2005 Bulletin 2005/10**

(73) Titulaire: **Ecole Nationale Supérieure d'Arts et Métiers ENSAM**
**75013 Paris (FR)**

(72) Inventeurs:
• **SKALLI, Wafa**
**F-75013 Paris (FR)**

• **DUMAS, Raphael**
**F-69100 Villeurbanne (FR)**
• **MITTON, David**
**F-94270 Kremlin Bicetre (FR)**
• **BATAILLE, Philippe**
**F-75002 Paris (FR)**
• **QUIDET, Damien**
**F-77100 Meaux (FR)**

(74) Mandataire: **Novagraaf Technologies**
**122 rue Edouard Vaillant**
**92593 Levallois-Perret Cedex (FR)**

(56) Documents cités:
| | |
|---|---|
| **WO-A1-01/50956** | **FR-A- 2 720 930** |
| **US-A- 4 719 646** | **US-A- 6 044 132** |
| **US-A- 6 055 449** | **US-A1- 2001 039 421** |

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**EP 1 511 421 B1**

**Description**

La présente invention concerne un dispositif de stéréoradiographie permettant le calibrage des radiographies qui constitue le pré-requis indispensable à la reconstruction d'une image en trois dimensions à partir de deux clichés obtenus selon deux dimensions et en particulier un dispositif de stéréoradiographie destiné à positionner le sujet à radiographier et à être interposé entre une source de rayons X et un récepteur desdits rayons, comprenant un plateau horizontal mobile en rotation et/ou en translation selon une seule direction. La présente invention concerne également le procédé de calibrage et d'utilisation du présent dispositif.

Il existe de nombreuses publications et brevets qui divulguent la possibilité de reconstruire un objet en trois dimensions à partir de clichés obtenus en deux dimensions. C'est en particulier le cas de la stéréovision. La stéréoradiographie, ou radiographie multiplanaire, est basée sur les mêmes principes, la troisième dimension est donnée par la mise en correspondance d'au moins deux vues prises sous des incidences différentes et cette mise en correspondance n'est pas possible sans une étape de calibrage qui, en stéréovision est le plus souvent effectuée une fois pour toute avant la prise des images. Dans le cadre de la radiographie, ces différentes incidences imposent soit l'utilisation de plusieurs sources de rayons X, soit un déplacement spatial d'une source unique ou un déplacement du sujet à radiographier devant cette source unique.

Or, dans le cadre de la radiographie clinique, l'emploi simultané de deux sources de rayons X est extrêmement rare. De plus, la position de la source de rayons X doit être réglée pour chaque sujet à radiographier.

Un exemple en est la représentation en trois dimensions de la colonne vertébrale humaine. Les informations structurelles de position et morphométriques que de telles représentations proposent sont particulièrement intéressantes. Elles peuvent par exemple aider un chirurgien dans son diagnostic pré- et post-opératoire ou encore être de nature à favoriser les interventions chirurgicales assistées par ordinateur.

Les seules informations de ce type qu'un chirurgien peut facilement recueillir proviennent des systèmes stéréoradiographiques qui ne sont disponibles que dans un très petit nombre d'hôpitaux. L'appareillage est généralement constitué d'une source et d'un film radiographique et d'une table tournante sur laquelle est disposé le patient de manière à obtenir plusieurs vues sous des incidences différentes. Un objet de calibrage est également ajouté à l'appareillage.

Il est en effet nécessaire de calibrer ces vues de manière à identifier les paramètres géométriques de l'environnement radiographique, c'est-à-dire préciser les positions relatives de la source de rayons X par rapport au film.

Il existe de nombreuses méthodes de calibrage mais celles-ci reposent sur la description des positions relatives des images et des sources X (ou des caméras) dans le cas général. Pour déterminer l'environnement radiographique, ces méthodes nécessitent de gérer 18 variables lors des calculs. Dans ce cas, l'objet de calibrage doit entourer la totalité du volume de mesure ce qui le rend particulièrement volumineux et encombrant.

De plus, le patient doit aussi être placé dans ce volume de mesure et peut être mis dans un inconfort et un état de stress dus à la structure de l'objet de calibrage qui l'entoure et/ou à la longueur du processus de mesure et d'acquisition.

Il a également été proposé de placer le patient allongé sur une table, comme pour le scanner. Cependant, à la différence de la position debout, cette mesure n'est pas effectuée sous charge et les courbures de la colonne verticale (physiologiques ou pathologiques) en sont modifiées.

De plus, pour obtenir deux vues sous des incidences différentes avec un seul système générateur d'images, on peut avoir recours à une translation de la source (verticale ou horizontale). Mais les deux incidences obtenues sont souvent proches, ce qui engendre plus d'erreurs dans la reconstruction 3D. De plus les clichés ainsi obtenus ne correspondent pas aux radiographies courantes utilisées en routine clinique.

Le document WO 01/50956 A1 divulgue un dispositif de radiograhpie pour l'évaluation de la position d'équilibre du corps humain. Le préambule de La revendication 1 est basé sur son contenu.

Le document US 2001/0039421 a décrit des méthodes de calibrage et de reconstruction 3D à partir d'images 2D.

Les inventeurs ont donc mis au point, ce qui fait l'objet de l'invention, un dispositif interposé entre la source de rayons X et le récepteur vertical desdits rayons, permettant de placer le sujet dans deux positions consécutives en maintenant le sujet dans une position stable quasi immobile et permettant de faire apparaître dans les radiographies obtenues un ensemble de marqueurs dont la configuration est spécifique et les positions relatives dans l'espace sont connues et permettant une méthode de calibrage explicite pour le calcul de l'environnement radiographique de manière à faciliter les calculs de la reconstruction d'une image en trois dimensions à partir de deux radiographies calibrées.

A cet effet, le dispositif de stéréoradiographie destiné à positionner un sujet à radiographier et à être interposé entre une source de rayons X et un récepteur vertical desdits rayons, comprenant un plateau horizontal mobile en rotation et/ou en translation unidirectionnelle selon l'invention se caractérise en ce que ledit plateau est orientable selon deux positions référencées selon un angle de 90° l'une par rapport à l'autre et permettant la prise de deux clichés, correspondant à deux positions du sujet orthogonales l'une par rapport à l'autre et en ce que le dispositif comprend également une armature solidaire dudit plateau horizontal et éventuellement un dispositif complémentaire de maintien destiné à accueillir ledit sujet et à le maintenir dans une position stable, et au moins trois marqueurs en matériau radio

2

opaque disposés solidairement sur ladite armature, l'un desdits marqueurs étant identifié comme origine d'un repère tridimensionnel orthogonal, les autres marqueurs étant disposés de manière à fournir par projection sur les axes dudit repère la connaissance d'au moins une valeur de distance entre lesdits marqueurs selon les trois axes X, Y, et Z dudit repère, respectivement Lx, Ly et H.

En outre, grâce au plateau tournant du dispositif, on obtient alors deux clichés successifs face et profil qui pourront également être utilisés en tant que tel par le chirurgien pour un simple examen de routine.

De plus, la disposition d'un dispositif de maintien constitué par l'armature et/ou par un élément complémentaire, comme par exemple une paire de poignées, permet au sujet de trouver une position debout stable une fois monté sur ledit plateau, ce qui permet de maintenir le patient dans une position sensiblement identique lors de la rotation du plateau tournant pour l'amener de sa première position à la deuxième position après une rotation de 90° autour d'un axe central du plateau.

On obtient ainsi des radiographies calibrées puisqu'elles sont prises sous deux incidences, avec le sujet le plus immobile possible et contenant des marqueurs permettant le calcul de l'environnement radiographique, c'est à dire les paramètres décrivant la position et l'orientation relative du film et de la source lors de la prise des deux clichés.

Les clichés sont donc obtenus selon deux axes virtuels différents de prise de vue.

Le choix angulaire de rotation du plateau est à la fois dicté par des contraintes pratiques pour que les radiographies en tant que telles puissent être utilisées par le praticien mais également pour la simplification des calculs des images X projetées à partir d'un sujet. De plus, de nombreux invariants sont présents : la distance entre le récepteur et la source reste constante, de même la hauteur de la source par rapport au dispositif est également invariante par construction.

L'ensemble des marqueurs, qui constitue le moyen de calibrage, est placé sur le plateau tournant. Cet ensemble constitue alors par projection des lignes verticales et horizontales qui encadrent les radiographies du sujet.

Les paramètres de calibrage sont alors calculés plan par plan et vue par vue à partir d'un sous-ensemble de marqueurs (parmi ceux visibles dans les radiographies) contrairement à toutes les autres méthodes qui intègrent le calcul de l'ensemble des points constitutifs du volume de l'objet de calibrage. Les paramètres déjà calculés ou les invariants sont alors réutilisés à chaque étape.

De préférence, les marqueurs seront situés dans des plans horizontaux et verticaux du repère tridimensionnel dont le premier marqueur est l'origine.

Dans une variante de réalisation du dispositif selon l'invention, celui-ci comprend trois marqueurs en matériau radio opaque, le premier marqueur étant identifié comme origine dudit repère, le second marqueur étant disposé dans un plan vertical comprenant ledit premier marqueur et à une hauteur H connue par rapport audit premier marqueur et à une distance Ly par rapport à l'axe vertical dudit repère et le troisième marqueur étant situé dans un plan horizontal passant par ledit premier marqueur et à l'aplomb dudit premier marqueur et à une distance Lx par rapport audit premier marqueur.

Dans une autre variante de réalisation, le dispositif selon l'invention comprend six marqueurs en matériau radio opaque, le premier marqueur étant identifié comme origine dudit repère, quatre marqueurs étant disposés dans un plan vertical comprenant ledit premier marqueur et formant deux paires de marqueurs ou dipôles, chacun des dipôles étant disposé selon la verticale, le premier dipôle définissant une droite passant par ledit premier marqueur, le second dipôle étant situé à une distance Ly par rapport audit premier dipôle, les deux marqueurs de chaque dipôle étant séparés d'une hauteur H et le sixième marqueur étant situé dans un plan horizontal passant par ledit premier marqueur et à l'aplomb dudit premier marqueur et à une distance Lx par rapport audit premier marqueur

Enfin, dans une dernière variante de réalisation du dispositif selon l'invention, celui-ci comprend plus de six marqueurs en matériau radio opaque disposés selon deux lignes verticales parallèles et deux lignes horizontales perpendiculaires aux dites lignes verticales et l'un desdits marqueurs est identifié comme origine dudit repère.

L'utilisation d'un nombre plus élevé de marqueurs, mais toujours disposés sur des lignes selon les axes du repère permet de rendre l'information $L_X$, $L_Y$, H redondante.

De manière préférentielle, ledit marqueur identifié comme origine dudit repère tridimensionnel comporte un marquage particulier en matériau radio opaque permettant de le distinguer simplement des autres marqueurs.

En particulier, ledit matériau radio opaque est un acier.

Le dispositif selon l'invention représente un système intégré qui permet à la fois le positionnement du patient, la projection des marqueurs sur les films et la rotation de l'ensemble pour obtenir deux clichés. Ce protocole est alors parfaitement compatible avec le protocole clinique de prise de radiographies (face, profil et source fixe).

Ce dispositif peut bien entendu fonctionner avec tout type de source de rayonnement X utilisée dans les applications usuelles et notamment dans le milieu médical et tout récepteur de rayons X, comme par exemple, les porte films argentiques classiques mais encore les détecteurs de rayons X numériques ou tout autre dispositif connu ou inconnu à ce jour.

Ce dispositif s'avère d'une utilisation simple pour les manipulateurs, par exemple les radiologues, et ne nécessite pas d'autre réglage que ceux de la source et du récepteur comme dans la prise de radiographie classique.

La structure du dispositif est ouverte, facile d'accès particulièrement dans le domaine médical pour un patient qui se positionne simplement en se maintenant à l'aide de l'armature et/ou d'un dispositif de maintien complémentaire éventuellement disposé à cet effet.

Les projections des marqueurs pourront de manière pratique être disposés sur les bords de la radiographie pour que le cliché soit le moins masqué possible d'où leur positionnement réel physique sur l'armature du plateau.

L'invention sera mieux comprise à la lecture de la description détaillée ci après faite en référence au dessin pour une application dans le cadre médical :

- La Figure 1 présente une variante de réalisation du dispositif selon l'invention ne comportant que trois marqueurs.
- La Figure 2 présente une autre variante de réalisation du dispositif selon l'invention comportant six marqueurs,
- La Figure 3 présente une autre variante de réalisation du dispositif selon l'invention comportant un grand nombre de marqueurs.
- La Figure 4 présente la variante de la figure 3 après rotation de 90°.

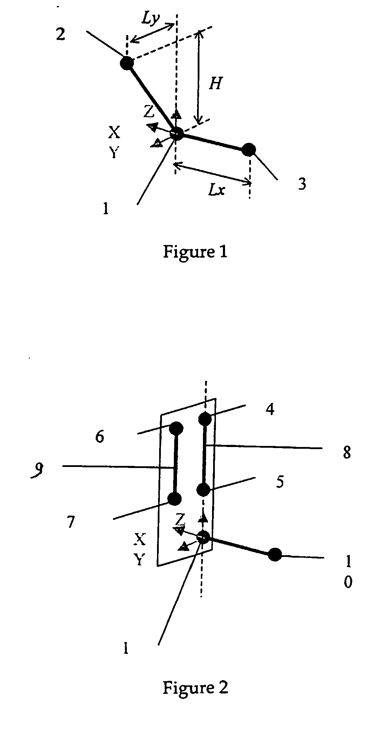

Sur la Figure 1, un marqueur 1 est identifié comme origine (0,0,0) d'un repère tridimensionnel en référence aux axes X, Y, et Z. Un second marqueur 2 est à la position définie par les coordonnées (a, Ly, H) où les valeurs Ly et H doivent être connues de l'utilisateur et où la valeur de a peut être quelconque. Un troisième marqueur 3 est à la position définie par les coordonnées (Lx, b, c) où la valeur Lx doit être connue par l'utilisateur et où les valeurs de b et c peuvent être quelconques.

Sur la figure 2, un marqueur 1 est identifié comme origine d'un repère tridimensionnel en référence aux axes X, Y, et Z. Quatre marqueurs 4, 5, 6 et 7 sont disposés dans un plan vertical comprenant le premier marqueur 1 et formant deux paires, 8 et 9, de marqueurs, chacune des paires 8 et 9 est disposée selon la verticale et la première paire 8 définit une droite passant par le premier marqueur 1. La seconde paire 9 est située à une distance Ly par rapport à la première paire 8. Les deux marqueurs 4 et 5, respectivement 6 et 7 de chaque paire 8 et 9 sont séparés d'une hauteur H et le cinquième marqueur 10 est situé dans un plan horizontal passant par le premier marqueur 1 et à l'aplomb de celui ci à une distance Lx par rapport audit premier marqueur 1. Ces distances sont fixées par construction et connues de l'utilisateur.

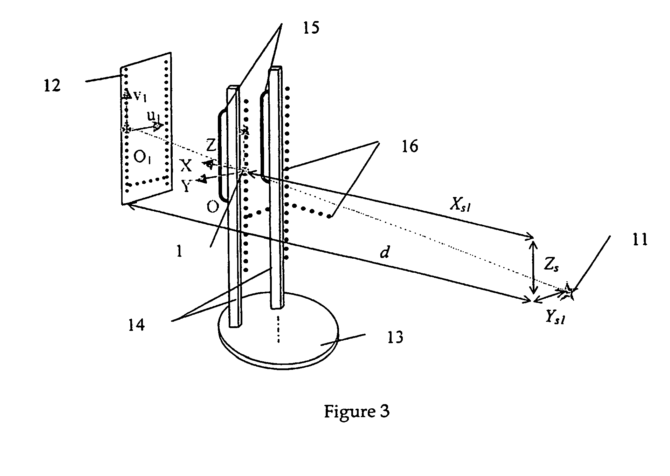

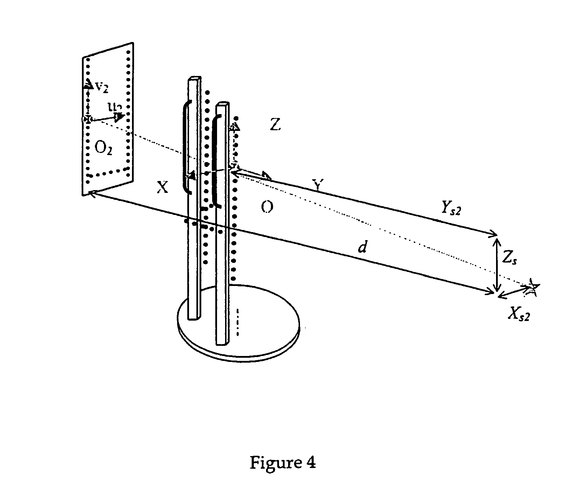

Sur les figures 3 et 4, le dispositif selon l'invention présente un ensemble de plus de six marqueurs. Ainsi, le dispositif est placé entre une source de rayons X 11 et un porte film vertical 12 pour collecter les rayons X émis par la source 11. Le dispositif comprend un plateau horizontal 13 mobile en rotation selon des moyens mécaniques usuels et orientable selon deux positions référencées selon un angle de 90° l'une par rapport à l'autre et permettant la prise de deux clichés orthogonaux l'un par rapport à l'autre en faisant simplement tourner le plateau 13 autour de son axe. Les positions 0 et 90 sont repérés en coopérant par exemple avec des taquets de blocage, des goupilles, des marquages de couleur ou tout autre dispositif de repérage ou de blocage (non représentés). Une armature 14 solidaire du plateau horizontal 13 et équipée ici d'une paire de poignées 15 disposée en tant que dispositif de maintien complémentaire éventuel destinées à accueillir un sujet tout en lui assurant un bon équilibre et une position de repos qu'il pourra conserver lors de la rotation du plateau 13 entre les deux positions de prise de vue. Des marqueurs 16 en matériau radio opaque sont disposés solidairement sur l'armature 14 selon au moins deux plans horizontal et vertical et ainsi les marqueurs forment un repère tridimensionnel dans lequel le marqueur 1 est identifié comme origine du repère, les autres marqueurs 16 étant disposés de manière à fournir la connaissance d'au moins une valeur de distance entre lesdits marqueurs 16 selon les trois axes X, Y et Z du repère, respectivement Lx, Ly et H.

Sur la figure 4, on présente le dispositif selon l'invention après rotation de 90° et en position pour effectuer le second cliché.

L'utilisation du dispositif selon l'invention se caractérise en ce que l'on place ledit dispositif entre ladite source de rayon X 11 et ledit récepteur de rayons X 12 de manière à ce que le plan vertical dudit repère tridimensionnel soit parallèle au plan dudit récepteur 12, en ce que l'on place le patient sur ledit plateau tournant 13, en ce que le patient se maintient dans une position de repos stable et figée à l'aide desdites poignées 15, on effectue un premier cliché du patient dans cette position, en ce que l'on effectue une rotation dudit plateau tournant 13 et éventuellement une translation de dégagement ou d'engagement du dispositif au plus près du film, ladite rotation étant de 90° et entraînant solidairement ladite armature 14, lesdits marqueurs 1,16 et ledit patient et en ce que l'on effectue un deuxième cliché dudit patient.

L'utilisation du dispositif se révèle alors particulièrement simple à la fois pour le praticien et le patient qui est placé dans une position de repos telle qu'il reste immobile durant toute la prise des clichés même lors de la rotation ou de la translation du plateau tournant 13.

La source de rayons X 11 est localisée en face du sujet (qui reste quasiment immobile sur le dispositif) puis le système est placé dans deux positions successives, indexées de telle manière à orienter les axes du repère de l'espace (X,Y,Z) respectivement parallèles et perpendiculaires au plan du film radiographique 12.

Les marqueurs radio-opaques 1,16 se projettent alors dans l'image radiographique selon la disposition suivante : le marqueur 1 identifié comme origine du repère et les lignes verticales sont visibles dans les deux images, une des deux lignes horizontales est visible dans l'une des deux images. Il s'agit de la ligne qui est parallèle au plan du film.

On appelle lignes verticales ou horizontales, les lignes obtenues en reliant les marqueurs 1,4,5,6,7,10,16 du système par exemple dans le cas d'un dispositif selon l'invention conçu sur le principe décrit et présenté aux figures 2 et 3, ou alors les projections des lignes reliant les différents marqueurs 1,2,3 du dispositif sur les axes du repère tridimensionnel, comme par exemple dans le cas d'un dispositif conçu sur le principe décrit et présenté à la figure 1.

De cette façon, un repère de référence pour chaque image radiographique peut être défini, $R_{image1}$ ($O_1$, $u_1$, $v_1$, $w_1$) figure 3 et $R_{image2}$ ($O_2$, $u_2$, $v_2$, $w_2$) figure 4 : à savoir l'origine fixée sur la projection du marqueur identifié comme origine du repère et les axes selon les projections des lignes verticales et horizontales.

Par conséquent, le dispositif de stéréoradiographie permet de déterminer des conditions spécifiques pour la prise des images radiographiques.

Premièrement, l'orientation du repère de référence de l'espace ($R_{espace}$) par rapport aux deux repères de référence des images ($R_{image1}$ et $R_{image2}$) est fixée et connue. Les relations géométriques entre les coordonnées tridimensionnelles et bidimensionnelles (images 1 et 2) sont établies en fonction de deux signes $\mathbf{m}_1$ et $\mathbf{m}_2$ connus, imposés par la position du système tournant. La matrice M de passage de $R_{espace}$ à $R_{image}$ est alors définie par :

$$M = \begin{bmatrix} m_1 & m_2 & 0 \\ 0 & 0 & 1 \\ m_2 & -m_1 & 0 \end{bmatrix}$$ dans laquelle les couples

($m_1$, $m_2$) = (1,0) pour la radiographie latérale droite

($m_1$, $m_2$) = (-1,0) pour la radiographie latérale gauche

($m_1$, $m_2$) = (0,-1) pour la radiographie postero-antérieure

($m_1$, $m_2$) = (0,1) pour la radiographie antero-postérieure

Ensuite, la distance entre le film radiographique 12 (d) et la source 11 ainsi que la hauteur de celle-ci par rapport au système ($Z_s$) reste inchangée pendant la prise successive des deux images, seul le dispositif est déplacé en rotation et éventuellement en translation.

Enfin, pour chaque cliché, la position de la source de rayons X 11 exprimée par rapport aux repères de référence des images ($u_{s1}$, $v_{s1}$, $u_{s2}$, $v_{s2}$ et d) et exprimée par rapport au repère de référence de l'espace ($X_{s1}$, $Y_{s1}$, $X_{s2}$, $Y_{s2}$ et $Z_s$) sont liées géométriquement du fait de la construction des repères en projection.

Ainsi, ces conditions spécifiques, imposées par le dispositif, permettent de définir l'environnement de la prise des images radiographiques avec un nombre d'inconnues géométriques indépendantes limité à six (au lieu de dix-huit dans le cas général).

Il est alors nécessaire de calibrer l'environnement radiographique des prises de vue, c'est-à-dire déterminer les positions relatives des images par rapport aux sources de rayons X, qui peuvent être décrites par 6 inconnues indépendantes (par exemple).

Le calcul des paramètres indépendants de l'environnement radiographique (susmentionnés ou autres) est effectué selon une procédure de calibrage spécifique en utilisant au mieux la configuration des marqueurs radio opaques (l'information projetée est différente d'une image à l'autre et selon les axes considérés) et en intégrant les invariants et les relations géométriques dues à la construction des repères en projection et citées ci-dessus.

En particulier, on peut calculer spécifiquement $X_{s1}$, $Y_{s1}$, $X_{s2}$, $Y_{s2}$, $Z_s$ et d, en tant que paramètre de l'environnement radiographique par une procédure spécifique, considérant séparément et successivement les plans verticaux et horizontaux des deux images.

Selon la configuration des marqueurs la procédure peut prendre la forme suivante :

La première étape de la procédure consiste à déterminer les paramètres liés au plan vertical de l'image 1 (d, $Z_s$ et $Y_{s1}$) en considérant les coordonnées des lignes de marqueurs verticales (exprimées dans $R_{espace}$) et leur projection (exprimées dans $R_{image1}$) .On utilise alors la méthode des moindres carrés avec les équations :

$$Y v_{s1} + v_1 Y_{s1} + \frac{Zd}{m_1 k} = v_1 Y \quad et \quad v_{s1} = -\frac{Z_s d}{m_1 k Y_{s1}}$$

La seconde étape de la procédure consiste à déterminer le paramètre lié au plan horizontal de l'image 1 ($X_{s1}$) en considérant les coordonnées des lignes de marqueurs horizontales (exprimées dans $R_{espace}$) et leur projection (exprimées dans $R_{image1}$) ainsi que les paramètres déjà calculés (d et $Y_{s1}$). On utilise ici encore la méthode des moindres

carrés sur :

$$Yu_{s1}+u_1Y_{s1}+Xd = u_1Y \text{ et } u_{s1} = -\frac{X_{s1}d}{Y_{s1}}$$

La troisième étape de la procédure consiste à déterminer le paramètre lié au plan vertical de l'image 2 ($X_{s2}$) en considérant les coordonnées des lignes de marqueurs verticales (exprimées dans $R_{espace}$) et leur projection (exprimées dans $R_{image2}$) ainsi que les paramètres déjà calculés (d et $Z_s$).

On utilise alors :

$$V_{s2} = \frac{Z_s d}{m_2 k X_{s2}} \text{ et } Xv_{s2} + v_2 X_{s2} + \frac{Zd}{m_2 k} = v_2 X$$

et on obtient :

$$v_2 X_{s2}^{2} + \left( \frac{Zd}{m_2 k} - v_2 X \right) X_{s2} - \frac{XZ_s d}{m_2 k} = 0$$

La quatrième et dernière étape de la procédure consiste à déterminer le paramètre lié au plan horizontal de l'image 2 ($Y_{s2}$) en considérant les coordonnées des lignes de marqueurs horizontales (exprimées dans $R_{espace}$) et leur projection (exprimées dans $R_{image2}$) ainsi que les paramètres déjà calculés (d et $X_{s2}$) .On utilise alors :

$$Xu_{s2} + u_2 X_{s2} - Yd = u_2 X \text{ et } u_{s2} = \frac{Y_{s2}d}{X_{s2}}$$

Le calcul des paramètres géométriques de l'environnement est effectué par un programme informatique dont les données d'entrée introduites sont les suivantes :

- L'orientation des radiographies prises sur le dispositif (postéro-antérieure ou antéro-postérieure et latérale droite ou latérale gauche).
- Les coordonnées des marqueurs dans chaque image. Ces coordonnées sont obtenues lors d'une étape d'identification de la projection des marqueurs dans le film, effectuée par un procédé standard (table à numériser ou logiciel informatique après numérisation des films radiographiques).
- Les coordonnées tridimensionnelles des marqueurs, obtenues une fois pour toutes lors de la fabrication du dispositif (données de fabrication, verniers, pieds à coulisses).

A la fin du programme, l'ensemble des paramètres géométriques de l'environnement est calculé par un algorithme approprié (les six paramètres indépendants plus les autres, déduits ou connus à priori).

La présente invention n'est bien entendu pas limitée aux variantes d'exécution présentées ci avant à titre d'exemples illustratifs non limitatifs ni aux variantes de mise en oeuvre du dispositif selon l'invention dans les procédés décrits.

Ainsi, il sera possible de combiner le dispositif selon l'invention avec par exemple une table de pression permettant de déterminer la ligne de gravité du corps et pouvoir utiliser cette information en corrélation avec celles que fournit directement le dispositif selon l'invention.

**Revendications**

1. Dispositif de stéréoradiographie destiné à positionner un sujet à radiographier et à être interposé entre une source de rayons X (11) et un récepteur vertical (12) desdits rayons, comprenant un plateau horizontal (13) mobile en rotation et éventuellement en translation unidirectionnelle, dans lequel ledit plateau (13) est orientable selon deux positions référencées selon un angle de 90° l'une par rapport à l'autre et permettant la prise de deux clichés correspondant à deux positions du sujet orthogonales l'une par rapport à l'autre et en ce que le dispositif comprend également une armature verticale (14) solidaire dudit plateau horizontal (13) et, éventuellement, un dispositif complémentaire de maintien (15) destiné à accueillir ledit sujet et à le maintenir dans une position stable, et au moins trois marqueurs (16) en matériau radio opaque l'un desdits marqueurs (1) étant identifié comme origine d'un repère tridimensionnel orthogonal, les autres marqueurs (16) étant disposés de manière à fournir par projection la connaissance d'au moins une valeur de distance entre lesdits marqueurs (16) selon les trois axes X, Y et Z dudit repère, respectivement Lx, Ly et H, **caractérisé en ce que** lesdits au moins trois marqeurs (16) sont disposés solidairement sur ladite armature (14).

2. Dispositif selon la revendication 1 **caractérisé en ce qu'**il comprend trois marqueurs en matériau radio opaque, le premier marqueur (1) étant identifié comme origine dudit repère, le second marqueur (2) étant disposé dans un plan vertical comprenant ledit premier marqueur et à une hauteur H connue par rapport audit premier marqueur et à une distance Ly par rapport à l'axe vertical dudit repère et le troisième marqueur (3) étant situé dans un plan horizontal passant par ledit premier marqueur et à l'aplomb dudit premier marqueur et à une distance Lx par rapport audit premier marqueur.

3. Dispositif selon la revendication 1 **caractérisé en ce qu'**il comprend six marqueurs en matériau radio opaque, le premier marqueur (1) étant identifié comme origine dudit repère, quatre marqueurs (4, 5, 6, 7) étant disposés dans un plan vertical comprenant ledit premier marqueur (1) et formant deux dipôles (8, 9), chacun des dipôles (8, 9) étant disposé selon la verticale, le premier dipôle (8) définissant une droite passant par ledit premier marqueur (1), le second dipôle (9) étant situé à une distance Ly par rapport audit premier dipôle (9), les deux marqueurs (4 et 5, 6 et 7) de chaque dipôle (8 et 9) étant séparés d'une hauteur H et le sixième marqueur (10) étant situé dans un plan horizontal passant par ledit premier marqueur (1) et à l'aplomb dudit premier marqueur (1) et à une distance Lx par rapport audit premier marqueur (1).

4. Dispositif selon la revendication 1 **caractérisé en ce qu'**il comprend plus de six marqueurs (16) en matériau radio opaque disposés selon deux lignes verticales parallèles et deux lignes horizontales perpendiculaires audites lignes verticales et **en ce que** l'un desdits marqueurs (1) est identifié comme origine dudit repère.

5. Dispositif selon l'une quelconque des revendications 1 à 4, **caractérisé en ce que** ledit marqueur (1) identifié comme origine dudit repère tridimensionnel comporte un marquage en matériau radio opaque permettant de le distinguer des autres marqueurs (16).

6. Dispositif selon l'une quelconque des revendications 1 à 4, **caractérisé en ce que** ledit matériau radio opaque est un acier.

7. Procédé d'utilisation du dispositif selon l'une des revendications 1 à 6, **caractérisé en ce que** l'on place ledit dispositif entre ladite source de rayon X (11) et ledit récepteur (12) de rayons X de manière à ce que un plan vertical dudit repère tridimensionnel soit parallèle au plan dudit récepteur (12), **en ce que** l'on place le patient sur ledit plateau tournant (13), **en ce que** le patient se maintient dans une position de repos stable et figée à l'aide de poignées (15), **en ce que** l'on effectue un premier cliché du patient dans cette position, **en ce que** l'on effectue une rotation dudit plateau tournant (13) et éventuellement une translation de dégagement ou d'engagement du dispositif au plus près du film, ladite rotation étant de 90° et entraînant solidairement ladite armature (14), lesdits marqueurs (1, 16) et ledit patient et **en ce que** l'on effectue un deuxième cliché dudit patient.

8. Procédé de calibrage du dispositif selon l'une quelconque des revendications 1 à 6, **caractérisé en ce que** l'on calcule spécifiquement 6 paramètres indépendants de l'environnement radiographique en utilisant au mieux la configuration des marqueurs radio-opaques et en intégrant les invariants et les relations géométriques dues à la construction des repères en projection.

9. Procédé selon la revendication 8, **caractérisé en ce que** l'on calcule spécifiquement les paramètres de l'environnement radiographique respectivement $X_{s1}$, $Y_{s1}$, $X_{s2}$, $Y_{s2}$, $Z_s$ et d en considérant séparément et successivement les plans verticaux et horizontaux des deux images.

10. Procédé selon l'une, quelconque, des revendications 8 et 9, **caractérisé en ce que** :

- l'on détermine les paramètres liés au plan vertical de la première image en considérant les coordonnées des lignes desdits marqueurs verticales et leur projection,
- l'on détermine le paramètre lié au plan horizontal de la première image en considérant les coordonnées des lignes de marqueurs horizontales et leur projection ainsi que les paramètres déjà calculés,
- l'on détermine le paramètre lié au plan vertical de la deuxième image en considérant les coordonnées des lignes de marqueurs verticales et leur projection ainsi que les paramètres déjà calculés et enfin,
- l'on détermine le paramètre lié au plan horizontal de la deuxième image en considérant les coordonnées des lignes de marqueurs horizontales et leur projection ainsi que les paramètres déjà calculés.

11. Procédé de traitement des radiographies comprenant une étape d'obtention de radiographies à l'aide du dispositif selon l'une quelconque des revendications 1 à 6 de manière à permettre le calibrage **caractérisé en ce que** sont introduites :

- l'orientation des radiographies prises sur ledit dispositif,
- les coordonnées des marqueurs dans chaque image par une identification de la projection des marqueurs dans le film,
- les coordonnées tridimensionnelles des marqueurs, obtenues lors de la fabrication du dispositif,

et **en ce que** l'on calcule par un algorithme approprié de l'ensemble des paramètres géométriques de l'environnement.

**Claims**

1. Stereoradiography device intended to position a subject to be X-rayed and to be interposed between an X-ray source (11) and a vertical receiver (12) for said rays, comprising a horizontal plate (13) able to move in rotation and optionally in unidirectional translation, wherein said plate (13) can be oriented in two referenced positions at an angle of 90° with respect to each other and enabling two shots to be taken corresponding to two positions of the subject orthogonal to each other and in that the device also comprises a vertical frame (14) secured to said horizontal plate (13) and, optionally, a complementary holding device (15) intended to accommodate said subject and to hold him in a stable position, and at least three markers (16) made from radio-opaque material, one of said markers (1) being identified as the origin of an orthogonal three-dimensional reference frame, the other markers (16) being placed so as to provide, by projection, knowledge of at least one distance value between said markers (16) along the three axis x, y and z of said reference frame, respectively Lx, Ly and H, **characterized in that** said at least three markers (16) are placed securely on said frame (14).

2. Device according to claim 1, **characterized in that** it comprises three markers made from radio-opaque material, the first marker (1) being identified as the origin of said reference frame, said second marker (2) being placed in a vertical plane comprising said first marker and at a height H known with respect to said first marker and at a distance Ly with respect to the vertical axis of said reference frame and the third marker (3) being situated in a horizontal plane passing through said first marker and vertically in line with said first marker and at a distance Lx with respect to said first marker.

3. Device according to claim 1, **characterized in that** it comprises six markers made from radio-opaque material, the first marker (1) being identified as the origin of said reference frame, four markers (4, 5, 6, 7) being placed in a vertical plane comprising said first marker (1) and forming two dipoles (8, 9), each of the dipoles (8, 9) being placed along the vertical, the first dipole (8) defining a straight line passing through said first marker (1), the second dipole (9) being situated at a distance Ly with respect to said first dipole (9), the two markers (4 and 5, 6 and 7) of each dipole (8 and 9) being separated by a height H and the sixth marker (10) being situated in a horizontal plane passing through said first marker (1) and vertically in line with said first marker (1) and at a distance Lx with respect to said first marker (1).

4. Device according to claim 1, **characterized in that** it comprises more than six markers (16) made from radio-opaque material placed along two parallel vertical lines and two horizontal lines perpendicular to said vertical lines and **in**

**that** one of said markers (1) is identified as the origin of said reference frame.

5. Device according to any one of claims 1 to 4, **characterized in that** said marker (1) identified as the origin of said three-dimensional reference frame comprises a marking in radio-opaque material enabling it to be distinguished from the other markers (16).

6. Device according to any one of claims 1 to 4, **characterized in that** said radio-opaque material is a steel.

7. Method of using the device according to one of claims 1 to 6, **characterized in that** said device is placed between said X-ray source (11) and said X-ray receiver (12) so that a vertical plane of said three-dimensional reference frame is parallel to the plane of said receiver (12), **in that** the patient is placed on said rotating plate (13), **in that** the patient holds himself in a stable position of rest fixed by means of handles (15), **in that** a first shot of the patient is effected in this position, **in that** a rotation of said rotating plate (13) is effected and optionally a disengagement or engagement translation of the device as close as possible to the film, said rotation being 90° and integrally driving said frame (14), said markers (1, 16) and said patient, and **in that** a second shot of said patient is effected.

8. Method of calibrating the device according to any one of claims 1 to 6, **characterized in that** 6 independent parameters of the radiographic environment are specifically calculated by best using the configuration of the radio-opaque markers and integrating the invariants and the geometric relationships due to the construction of the reference frames in projection.

9. Method according to claim 8, **characterized in that** the parameters of the radiographic environment respectively $X_{s1}$, $Y_{s1}$, $X_{s2}$, $Y_{s2}$, $Z_s$ and d are specifically calculated by considering separately and successively the vertical and horizontal planes of the two images.

10. Method according to either one of claims 8 and 9, **characterized in that**:

    - the parameters relating to the vertical plane of the first image are determined by considering the coordinates of the lines of said vertical markers and their projection,
    - the parameter relating to the horizontal plane of the first image is determined by considering the coordinates of the lines of horizontal markers and the projection thereof as well as the parameters already calculated,
    - the parameter relating to the vertical plane of the second image is determined by considering the coordinates of the lines of vertical markers and the projection thereof as well as the parameters already calculated, and finally
    - the parameter relating to the horizontal plane of the second image is determined by considering the coordinates of the lines of horizontal markers and the projection thereof as well as the parameters already calculated.

11. Method of processing radiographs comprising a stage of obtaining radiographs by means of the device according to any one of claims 1 to 6 so as to allow calibration, **characterized in that** the following are introduced:

    - the orientation of the radiographs taken on said device,
    - the coordinates of the markers in each image by an identification of the projection of the markers in the film,
    - the three-dimensional coordinates of the markers, obtained during the manufacture of the device,

    and **in that** all the geometric parameters of the environment are calculated by means of a suitable algorithm.

**Patentansprüche**

1. Stereoröntgenvorrichtung zum Positionieren eines mit Röntgenstrahlen zu durchleuchtenden Objekts und zum Einfügen zwischen einer Röntgenstrahlenquelle (11) und einem vertikalen Empfänger (12) der Strahlen, umfassend eine horizontale Platte (13), die drehbar und gegebenenfalls in eine Richtung verschiebbar ist, wobei die Platte (13) gemäß zwei Referenzpositionen, die sich gemäß einem 90°-Winkel zueinander angeordnet befinden, schwenkbar ist und die Aufnahme von zwei Bildern ermöglicht, die zwei zueinander senkrecht stehenden Positionen des Objekts entsprechen, und wobei die Vorrichtung ebenfalls einen vertikalen Rahmen (14), der mit der horizontalen Platte (13) fest verbunden ist, und eventuell eine zusätzliche Haltevorrichtung (15), die zum Aufnehmen des Objekts und zu dessen Festhalten in einer stabilen Position dient, und wenigstens drei Marker (16) aus strahlenundurchlässigem Material umfasst, wobei einer der Marker (1) als Ausgangspunkt eines dreidimensionalen, orthogonalen Koordinatensystems identifiziert wird, wobei die anderen Marker (16) so angeordnet sind, dass sie durch Projektion die Kenntnis wenigstens eines Abstandswertes zwischen den Markern (16) gemäß den drei Achsen X, Y und Z des Koordinatensystems, also Lx, Ly und H, liefern, **dadurch gekennzeichnet, dass** die wenigstens drei Marker (16) fest am Rahmen (14) angeordnet sind.

2. Vorrichtung nach Anspruch 1, **dadurch gekennzeichnet, dass** sie drei Marker aus strahlenundurchlässigem Material umfasst, wobei der erste Marker (1) als Ausgangspunkt des Koordinatensystems identifiziert wird, wobei der zweite Marker (2) in einer vertikalen Ebene, die den ersten Marker umfasst, und in einer Höhe H, die in Bezug auf den ersten Marker bekannt ist, und in einem Abstand Ly in Bezug auf die vertikale Achse des Koordinatensystems angeordnet ist, und wobei sich der dritte Marker (3) in einer horizontalen Ebene befindet, die durch den ersten Marker hindurch und lotrecht zum ersten Marker und in einem Abstand Lx in Bezug auf den ersten Marker verläuft.

3. Vorrichtung nach Anspruch 1, **dadurch gekennzeichnet, dass** sie sechs Marker aus strahlenundurchlässigem Material umfasst, wobei der erste Marker (1) als Ausgangspunkt des Koordinatensystems identifiziert wird, wobei vier Marker (4, 5, 6, 7) in einer vertikalen Ebene, die den ersten Marker (1) umfasst, angeordnet sind und zwei Dipole (8, 9) bilden, wobei jeder der Dipole (8, 9) gemäß der Vertikalen angeordnet ist, wobei der erste Dipol (8) eine Gerade definiert, die durch den ersten Marker (1) hindurch verläuft, wobei sich der zweite Dipol (9) in einem Abstand Ly in Bezug auf den ersten Dipol (9) befindet, wobei die zwei Marker (4 und 5, 6 und 7) jedes Dipols (8 und 9) durch eine Höhe H getrennt sind, und wobei sich der sechste Marker (10) in einer horizontalen Ebene befindet, die durch den ersten Marker (1) hindurch und lotrecht zum ersten Marker (1) und in einem Abstand Lx in Bezug auf den ersten Marker (1) verläuft.

4. Vorrichtung nach Anspruch 1, **dadurch gekennzeichnet, dass** sie mehr als sechs Marker (16) aus strahlenundurchlässigem Material umfasst, die gemäß zwei parallelen, vertikalen Linien und zwei zu den vertikalen Linien senkrecht verlaufenden, horizontalen Linien angeordnet sind, und dadurch, dass einer der Marker (1) als Ausgangspunkt des Koordinatensystems identifiziert wird.

5. Vorrichtung nach einem der Ansprüche 1 bis 4, **dadurch gekennzeichnet, dass** der Marker (1), der als Ausgangspunkt des dreidimensionalen Koordinatensystems identifiziert wird, eine Markierung aus strahlenundurchlässigem Material umfasst, die es ermöglicht, ihn von den anderen Markern (16) zu unterscheiden.

6. Vorrichtung nach einem der Ansprüche 1 bis 4, **dadurch gekennzeichnet, dass** das strahlenundurchlässige Material ein Stahl ist.

7. Verfahren zur Verwendung der Vorrichtung nach einem der Ansprüche 1 bis 6, **dadurch gekennzeichnet, dass** die Vorrichtung zwischen der Röntgenstrahlenquelle (11) und dem Empfänger (12) der Röntgenstrahlen angeordnet wird, so dass eine vertikale Ebene des dreidimensionalen Koordinatensystems parallel zur Ebene des Empfängers (12) verläuft, dadurch, dass der Patient auf der Drehplatte (13) platziert wird, dadurch, dass sich der Patient mit Hilfe von Griffen (15) in einer stabilen und starren Ruheposition hält, dadurch, dass ein erstes Bild vom Patienten in dieser Position aufgenommen wird, dadurch, dass eine Drehung der Drehplatte (13) und eventuell eine Verschiebung der Vorrichtung von der Folie weg oder möglichst nahe zu dieser hin ausgeführt wird, wobei die Drehung 90° beträgt und sowohl den Rahmen (14) als auch die Marker (1, 16) und den Patienten als Einheit mit sich zieht, und dadurch, dass ein zweites Bild vom Patienten aufgenommen wird.

8. Verfahren zum Kalibrieren der Vorrichtung nach einem der Ansprüche 1 bis 6, **dadurch gekennzeichnet, dass** eigens 6 Parameter berechnet werden, die von der Röntgenumgebung unabhängig sind, indem die Konfiguration der strahlenundurchlässigen Marker bestmöglich herangezogen wird und indem die Invarianten und die geometrischen Beziehungen, die auf der Konstruktion der projizierten Koordinatensysteme gründen, integriert werden.

9. Verfahren nach Anspruch 8, **dadurch gekennzeichnet, dass** eigens die Parameter der Röntgenumgebung, also $X_{s1}$, $Y_{s1}$, $X_{s2}$, $Y_{s2}$, $Z_s$ und d berechnet werden, indem die vertikalen und horizontalen Ebenen der zwei Bilder getrennt und aufeinander folgend herangezogen werden.

10. Verfahren nach einem der Ansprüche 8 und 9, **dadurch gekennzeichnet, dass**:

    - die Parameter in Verbindung mit der vertikalen Ebene des ersten Bildes durch Heranziehen der Koordinaten der Linien der vertikalen Marker und ihrer Projektion bestimmt werden,
    - der Parameter in Verbindung mit der horizontalen Ebene des ersten Bildes durch Heranziehen der Koordinaten der Linien der horizontalen Marker und ihrer Projektion sowie der bereits berechneten Parameter bestimmt wird,

- der Parameter in Verbindung mit der vertikalen Ebene des zweiten Bildes durch Heranziehen der Koordinaten der Linien der vertikalen Marker und ihrer Projektion sowie der bereits berechneten Parameter bestimmt wird, und schließlich
- der Parameter in Verbindung mit der horizontalen Ebene des zweiten Bildes durch Heranziehen der Koordinaten der Linien der horizontalen Marker und ihrer Projektion sowie der bereits berechneten Parameter bestimmt wird.

11. Verfahren zur Verarbeitung der Röntgenbilder, umfassend den Schritt des Erhaltens von Röntgenbildern mit Hilfe der Vorrichtung nach einem der Ansprüche 1 bis 6, um die Kalibrierung zu ermöglichen, **dadurch gekennzeichnet, dass** Folgendes eingesetzt wird:

- die Ausrichtung der auf der Vorrichtung aufgenommenen Röntgenbilder,
- die Koordinaten der Marker in jedem Bild durch eine Identifikation der Projektion der Marker in der Folie,
- die dreidimensionalen Koordinaten der Marker, die man bei der Herstellung der Vorrichtung erhält,

und dadurch, dass mit Hilfe eines geeigneten Algorithmus die Gesamtheit der geometrischen Parameter der Umgebung berechnet wird.

Figure 1

Figure 2

Figure 3

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0150956 A1
- US 20010039421 A